# EUROPÄISCHE PATENTSCHRIFT

(11) **EP 2 194 984 B1**
(45) Veröffentlichungstag und Bekanntmachung des Hinweises auf die Patenterteilung: **09.01.2013**
(21) Anmeldenummer: 08801597.9
(22) Anmeldetag: 18.08.2008
(51) Int. Cl.: A61K 31/455, A61P 13/12

(54) **ARZNEIMITTEL ZUR BEHANDLUNG ODER PRÄVENTION VON PROTEINURIE**
MEDICAMENT FOR TREATING OR PREVENTING PROTEINURIA
MÉDICAMENT POUR LE TRAITEMENT OU LA PRÉVENTION DE LA PROTÉINURIE

(30) Priorität: 13.09.2007 DE 102007045038
(43) Veröffentlichungstag der Anmeldung: 16.06.2010
(73) Patentinhaber: Medice Arzneimittel Pütter GmbH & Co. KG, 58638 Iserlohn (DE)
(72) Erfinder: LANG, Florian, 72076 Tuebingen (DE)
(74) Vertreter: Witte, Weller & Partner
(86) Internationale Anmeldenummer: PCT/EP2008/006753
(87) Internationale Veröffentlichungsnummer: WO 2009/036852

(56) Entgegenhaltungen:
- EP-A- 0 219 276
- EP-A- 0 820 703
- FR-A- 2 703 248
- OWADA AKIRA ET AL: "Antiproteinuric effect of niceritrol, a nicotinic acid derivative, in chronic renal disease with hyperlipidemia: A randomized trial." AMERICAN JOURNAL OF MEDICINE, Bd. 114, Nr. 5, 1. April 2003 (2003-04-01), Seiten 347-353, XP002504074 ISSN: 0002-9343
- "Proteinurie" In: Pschyrembel: "Klinisches Wörterbuch", 1 January 2002 (2002-01-01), Walter de Gruyter, Berlin, New York vol. 259, page 1368,

## Beschreibung

Die vorliegende Erfindung betrifft ein Arzneimittel zur Behandlung und/oder Prävention von Proteinurie, ein Verfahren zur Herstellung eines solchen Arzneimittels sowie ein Verfahren zur therapeutischen und/oder prophylaktischen Behandlung eines Lebewesens, das von Proteinurie betroffen ist und/oder bei dem die Gefahr von Proteinurie besteht.

Unter Proteinurie, die im deutschen Sprachraum auch als "Eiweißharn" bezeichnet wird, versteht man die erhöhte Ausscheidung von Serumproteinen im Urin. Eine Proteinurie liegt vor, wenn die Eiweißausscheidung über den Urin über 200 bis 300 mg pro Tag beträgt. Ein kritischer Schwellenwert für die Diagnose einer Proteinurie stellt dabei ferner eine Konzentration von 150 mg Eiweiß in einem Liter Harn dar. Bei einem Überschreiten dieser Konzentration wird ebenfalls von Proteinurie gesprochen.

Bei den ausgeschiedenen Serumproteinen handelt es sich insbesondere um Albumin und Transferrin, niereneigene Proteine und Proteine der ableitenden Harnwege.

Bei den meisten Menschen findet sich bei einem einmaligem Auftreten einer Proteinurie keine Grunderkrankung. Bei häufigem oder chronischem Auftreten von Proteinurie wird oft eine Schädigung der Glomeruli der Niere festgestellt, so dass man von glomerulärer Proteinurie spricht. Findet man eine Schädigung der Tubuli der Niere, spricht man von tubulärer Proteinurie.

So zeigt sich, dass die Ursachen der Proteinurie äußerst vielfältig sind. Der glomerulären Proteinurie kann bspw. ein Diabetes mellitus zugrunde liegen. Weitere Ursachen können in einer arteriellen Hypertonie, Entzündungen der Niere, Medikamenten, EPH-Gestose in der Schwangerschaft, erblichen Schädigungen oder Stress bzw. zu hohen oder zu niedrigen Körpertemperaturen liegen.

Ursachen der tubulären Proteinurie können Entzündungen im Bereich der Tubuli, zu hohe Harnsäurewerte (Gicht), ein längerer Schmerzmittelmissbrauch, multiples Myelom, Störungen der Harnblasenentleerung mit Rückstau in der Niere, Hypokaliämie oder Hypercalcämie sein.

Die Ursachen können jedoch auch "stromaufwärts" der Niere lokalisiert sein. Dabei liegt meist ein Überangebot eines bestimmten Eiweißes im Blut vor, wie im Falle einer Hämolyse, Myoglobinämie oder eines Plasmocytoms mit der Entstehung von Bence-Jones-Proteinen.

Die Ursachen können ferner "stromabwärts" der Niere lokalisiert sein, beispielsweise in Form von Hamleitersteinen, Blasensteinen, Harnleiter- bzw. Blasenentzündungen oder Tumoren.

Die Proteinurie ist also nicht (nur) ein Faktor in der Progression einer Nierenerkrankung, sondern stellt ein eigenständiges Krankheitsbild dar.

Die Therapie einer Proteinurie beschränkt sich derzeit im Wesentlichen auf die Behandlung der Grunderkrankung, sofern eine solche bekannt ist, beispielsweise eine Diabetes mellitus oder einer Entzündung der Niere. Häufig ist die Behandlung der Grunderkrankung nicht hinreichend erfolgreich, um das Auftreten einer Proteinurie zu verhindern. Auch ist häufig die Grunderkrankung nicht bekannt, so dass keine zielgerichtete Therapie möglich ist.

In der FR 2 703 248 wird beschrieben, dass die Verabreichung einer Kombination aus 4-Hydroxybuttersäure und Nicotinsäureamid und Nicotinsäureamid allein den Blutzucker und die Ausscheidung von Zucker über den Urin reduziert. Es wird deshalb vorgeschlagen, eine Kombination aus 4-Hydroxybuttersäure und Nicotinsäureamid zur Behandlung von Diabetes mellitus einzusetzen.

Owada et al. (2003), The American Journal of Medicine, Vol. 114, Seiten 347-353, beschreiben, dass die Verabreichung von Niceritol die Proteinurie reduzieren kann.

Vor diesem Hintergrund ist es Aufgabe der Erfindung, ein Arzneimittel bereitzustellen, mit dem zielgerichtet eine Proteinurie behandelt oder dieser vorgebeugt werden kann.

Diese Aufgabe wird durch die Bereitstellung eines Arzneimittels gelöst, das als einzige gegen Proteinurie wirksame Substanz Nicotinsäureamid enthält.

Die Erfinder haben über Versuche an diabetischen Mäusen überraschenderweise festgestellt, dass durch die Verabreichung von Nicotinsäureamid die Ausscheidung von Serumproteinen, wie bspw. Albumin, über den Urin signifikant gehemmt werden kann.

Diese Erkenntnis der Erfinder war überraschend. Nicotinsäureamid, das auch als Nicotinamid, Niacinamid, Antipellagravitamin, Vitamin PP (engl. pellagra preventive factor), 3-Pyridincarboxamid (IUPAC), bezeichnet wird, ist ein Bestandteil des Vitamin-B2-Komplexes sowie von NAD(P). Nicotinsäureamid kommt in Leber, Fisch, Hefe und Getreidekeimlingen vor. Nicotinsäureamid weist ein Molekulargewicht von 122,12, die Summenformel C₆H₆N₂O und die CAS-Nummer 98-92-0 auf.

Nicotinsäureamid werden bislang völlig andere Eigenschaften zugeschrieben.

So beschreiben bspw. Eto et al.: Nicotinamide prevents the development of hyperphosphataemia by suppressing intestinal sodium-dependent phosphate transporter in rats with adenine-induced renal failure. Nephrol. Dial. Transplant. 2005; 20:1378-1384, dass Nicotinsäureamid in Ratten den natriumabhängigen Phosphattransporter NaPiIIb und damit die Aufnahme von Phosphat in den Organismus hemme. Die Autoren stellen die Hypothese auf, dass Nicotinsäureamid einen Schutz gegenüber einer Verschlechterung der Nierenfunktion bieten könne.

Tenenhouse H.S. und Chu Y.L.: Hydrolysis of nicotinamide-adenine dinucleotide by purified renal brush-border membranes. Mechanism of NAD+ inhibition of brush-border membrane phosphate-transport activity. Biochem. J. 1982; 204:635-638, behaupten ferner, dass Nicotinsäureamid die renale Resorption von Phosphat hemme und damit die renale Ausscheidung von Phosphat steigere.

Katai et al.: Nicotinamide inhibits sodium-dependent phosphate cotransport activity in red small intestine. Nephrol. Dial. Transplant. 1999; 14: 1195-1201, schlagen vor, dass Nicotinsäureamid die Na/Pi-Cotransportaktivität in den renalen proximalen Tubuli inhibieren könne.

Takahashi et al: Nicotinamide suppresses hyerphosphatemia in hemodialysis patients. Kidney International 2004; 65: 1099-1104, schlagen vor, dass Nicotinsäureamid zur Kontrolle von Hyperphosphatämie und Hyperparathyreoidismus in Dialysepatienten eingesetzt werden könnte.

Für Nicotinsäureamid ist ferner beschrieben worden, dass ein Mangel hiervon zum Krankheitsbild der sog. Pellagra führen kann, das sich als Dermatitis mit Hyperpigmentierung im Bereich sonnenexponierter Haut manifestiert. Die Verabreichung von Nicotinsäureamid wird deshalb zur Behandlung von Pellagra vorgeschlagen.

Verschiedene Autoren schlagen die Verabreichung von Nicotinsäureamid zur Verlängerung der Lebensdauer bzw. zur Verzögerung von Altersprozessen vor; vgl. Crane und Low: Plasma membrane redox and control of sirtuin. Age 2005; 27:147-152, Bitterman et al.: Inhibition of silencing and accelerated aging by nicotinamide, a putative negative regulator of yeast sir2 and human SIRT1. J. Biol. Chem. 2002; 277:45099-45107 (in Hefe), und Kang et al.: Nicotinamide extends replicative lifespan of human cells. Aging Cell 2006; 5:423-436 (in humanen Zellen).

In der nicht veröffentlichten DE 10 2007 003 524 wird außerdem die Verwendung von Nicotinsäureamid zur Behandlung von Arteriosklerose vorgeschlagen.

Die Verwendung von Nicotinsäureamid zur Behandlung und/oder Prävention von Proteinurie ist im Stand der Technik bislang nicht beschrieben.

Die der Erfindung zugrunde liegende Aufgabe wird somit durch die Bereitstellung von Nicotinsäureamid vollkommen gelöst.

Die Aufgabe wird ferner gelöst durch ein Verfahren zur Herstellung eines Arzneimittels zur Behandlung und/oder Prävention von Proteinurie, das die folgenden Schritte aufweist: (1) Bereitstellung von Nicotinsäureamid, und (2) Formulierung des Nicotinsäureamids in einem pharmazeutisch akzeptablen Träger.

Pharmazeutisch akzeptable Träger sind im Stand der Technik umfassend beschrieben. Beispielhaft wird verwiesen auf Bauer et al.: Lehrbuch der pharmazeutischen Technologie, 6. Aufl., Wissenschaftliche Verlagsgesellschaft mbH Stuttgart 1999; Rowe et al.: Handbook of pharmaceutical excipients 5. Aufl., Pharmaceutical Press and American Pharmacist Association 2006. Der Inhalt der vorstehend genannten Publikationen ist durch Inbezugnahme Bestandteil der Anmeldung.

Bei der erfindungsgemäßen Verwendung bzw. dem erfindungsgemäßen Verfahren ist es bevorzugt, wenn die Behandlung und/oder Prävention von Proteinurie bei einem Patienten erfolgt, der unter einer Krankheit/Störung leidet oder einer Veranlagung für eine Krankheit/Störung aufweist, die ausgewählt ist aus der Gruppe bestehend aus: chronisches Nierenversagen, Nierenentzündung, Harnleiterentzündung, Blasenentzündungen, Diabetes mellitus, arterielle Hypertonie, Medikamentenabhängigkeit, EPH-Gestose, erhöhte/erniedrigte Körpertemperatur, erhöhte Harnsäurewerte (Gicht), multiples Myelom, Störungen der Harnblasenentleerung mit Rückstau in der Niere, Hypokaliämie, Hypercalcämie, Hämolyse, Myoglobinämie, Tumoren, Plasmocytom mit der Entstehung von Bence-Jones-Proteinen, Harnleitersteine, Blasensteine.

Die vorstehend identifizierten Erkrankungen bzw. Komplikationen können sich in einer Proteinurie manifestieren. Die Verabreichung von Nicotinsäureamid an einen betroffenen Patienten hat deshalb den Vorteil, dass die nachteiligen Auswirkungen einer Proteinurie auf den Organismus eingeschränkt werden können.

Bei der erfindungsgemäßen Verwendung bzw. dem erfindungsgemäßen Verfahren ist es bevorzugt, wenn das Arzneimittel für eine Applikation ausgebildet ist, die ausgewählt ist aus der Gruppe bestehend aus: oral, rektal, parenteral, lokal, transdermal.

Diese Maßnahme hat den Vorteil, dass das Arzneimittel je nach gewünschter Applikationsform bereits auf geeignete Weise ausgestaltet ist. Dem behandelnden Arzt steht dadurch eine Auswahl verschiedenster Ausgestaltungsformen des Arzneimittels zur Verfügung, wobei sich die konkrete Form an dem Zustand des Patienten, dem jeweiligen Behandlungsprogramm und weiteren Faktoren orientieren kann.

Vor diesem Hintergrund ist es auch bevorzugt, wenn das Arzneimittel in einer Form ausgebildet ist, die ausgewählt ist aus der Gruppe bestehend aus: Pulver, Tablette, Saft, Tropfen, Kapsel, Zäpfchen, Lösung, Injektionslösung, Aerosol, Salbe, Spülung, Pflaster, Pellet, Dragee, modifiziert freisetzende Darreichungsform.

Mit dieser Maßnahme wird auf vorteilhafte Art und Weise bereits eine Formulierungsvariante bereitgestellt, die den unmittelbaren Einsatz von Nicotinsäureamid zur Behandlung bzw. Prävention von Proteinurie ermöglicht. Eine modifiziert freisetzende Darreichungsform, wie bspw. eine Retardform, ist besonders vorteilhaft, um eine gleichmäßige Freisetzung des Nicotinsäureamids im Organismus zu gewährleisten. Dies gilt umso mehr, da es Hinweise darauf gibt, dass Nicotinsäureamid vorwiegend im Duodenum resorbiert wird. Durch die Formulierung als modifiziert freisetzende Darreichungsform erreicht der Wirkstoff hingegen auch die hinteren Darmbereiche.

Bei der erfindungsgemäßen Verwendung bzw. dem erfindungsgemäßen Verfahren ist es außerdem bevorzugt, wenn das Arzneimittel Nicotinsäureamid in einer Konzentration aufweist, die ausgewählt ist aus der Gruppe bestehend aus (jeweils angegeben in Gewicht des Wirkstoffs Nicotinsäureamid bezogen auf das Gesamtgewicht des Arzneimittels): 1 µg/g, 10 µg/g, 100 µg/g, 1 mg/g, 10 mg/g, 100 mg/g, 1 g/g.

Diese Maßnahme hat den Vorteil, dass das Arzneimittel den Wirkstoff Nicotinsäureamid bereits in einer Konzentration aufweist, die sich zur Behandlung bzw. Prävention von Proteinurie besonders eignet.

Ferner ist es bevorzugt, wenn die Absolutmenge des Wirkstoffs Nicotinsäureamid in einer Dosierungseinheit des Arzneimittel zwischen ca. 1 und ca. 3000 mg, vorzugsweise zwischen ca. 10 und ca. 1500 mg, weiter bevorzugt zwischen ca. 100 und ca. 750 mg, und höchst bevorzugt bei ca. 500 mg liegt.

Mit dieser Maßnahme wird Nicotinsäureamid bereits in einer solchen Menge bereitgestellt, dass in dem Patient ohne weiteres die gewünschten Nicotinsäureamidspiegel erzielt werden können. Unter einer Dosierungseinheit wird erfindungsgemäß eine oben genannte Formulierungsvariante bezeichnet, bspw. eine Tablette, ein Dragee, eine Kapsel etc.

Weiter ist es bei der erfindungsgemäßen Verwendung bzw. dem erfindungsgemäßen Verfahren bevorzugt, wenn das Arzneimittel zusätzlich eine weitere gegen Proteinurie wirksame Substanz aufweist.

Diese Maßnahme hat den Vorteil, dass durch den Zusatz die Wirkungen des erfindungsgemäßen Arzneimittels gegen Proteinurie noch verbessert werden können. So ist es möglich, durch die Kombination mit einer weiteren Substanz einen Eingriff in die Proteinurie an einer anderen Stelle zu bewirken, um das Krankheitsbild durch synergistische Effekte noch wirksamer zu bekämpfen.

Bei der weiteren Substanz handelt es sich erfindungsgemäß vorzugsweise um Steroide oder nichtsteroidale Antiphlogistica.

Diese Maßnahme hat den Vorteil, dass das Arzneimittel eine solche weitere Substanz beinhaltet, die nachweislich eine Wirkung gegen Proteinurie zeigt und routinemäßig eingesetzt wird. Erfindungsgemäß können durch das Zusammenwirken von Nicotinsäureamid und der weiteren Substanz ggf. synergistische Effekte erzielt werden.

Vor diesem Hintergrund betrifft ein weiterer Gegenstand der vorliegenden Erfindung ein Verfahren zur therapeutischen und/oder prophylaktischen Behandlung eines Lebewesens, das von Proteinurie betroffen ist oder/und bei dem die Gefahr von Proteinurie besteht, das folgende Schritte aufweist: (1) Bereitstellung von Nicotinsäureamid, (2) Einbringung des Nicotinsäureamids in das Lebewesen, und (3) ggf. mehrfaches Wiederholen der Schritte (1) und (2).

Das Nicotinsäureamid kann bei diesem Verfahren in Form des erfindungsgemäßen Arzneimittels bereitgestellt und in das Lebewesen eingebracht werden. Die vorstehend beschriebenen Eigenschaften, Merkmale und Vorteile gelten insofern für dieses Verfahren entsprechend.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nun anhand von Ausführungsbeispielen näher erläutert, aus denen sich weitere Eigenschaften, Merkmale und Vorteile ergeben. Dabei wird Bezug genommen auf die beigelegten Figuren, in denen Folgendes dargestellt ist:

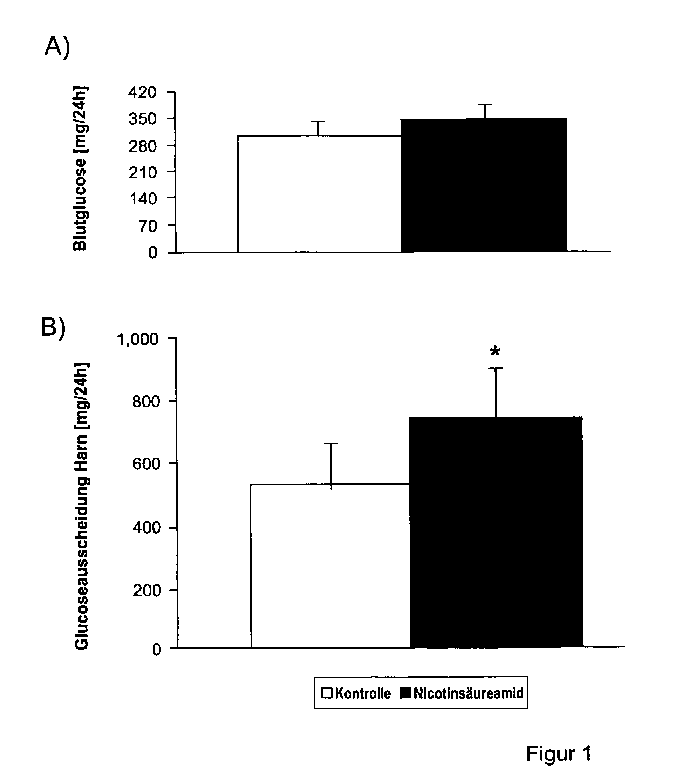

- Fig. 1: zeigt die Glucosekonzentration im Plasma und die Glucoseausscheidung über den Harn vor und nach der Verabreichung von Nicotinsäureamid; arithmetische Mittel ± SEM (n = 9) der (A) Glucosekonzentration im Plasma (mg/dl) und (B) der Glucoseausscheidung über den Harn (mg/24 h) vor (unausgefüllte Balken) und nach (ausgefüllte Balken) der Verabreichung von Nicotinsäureamid (0,5 mg/g, das dem Trinkwasser hinzugegeben wurde);

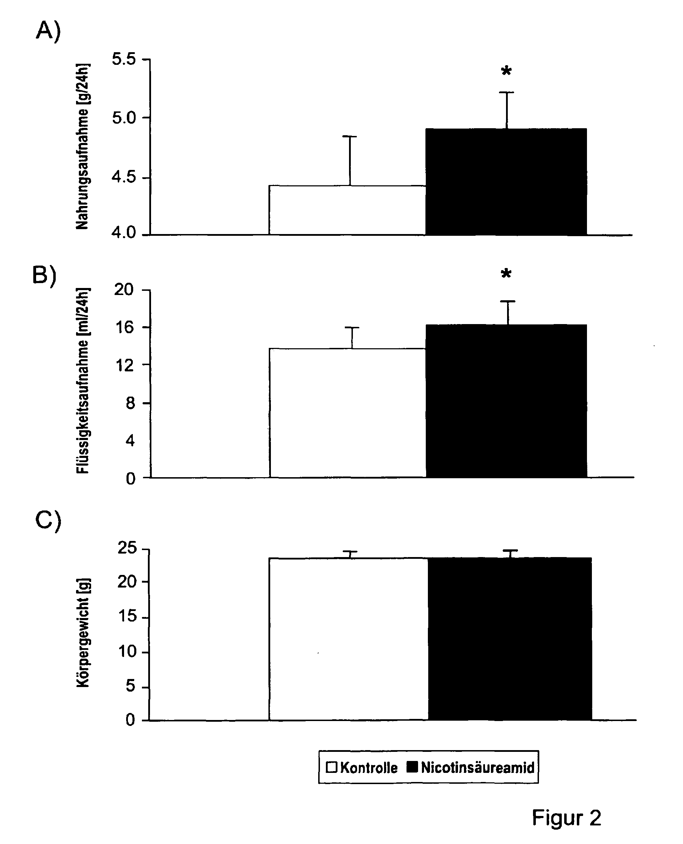

- Fig. 2: zeigt die Nahrungs- und Flüssigkeitsaufnahme als auch das Körpergewicht vor und nach der Verabreichung von Nicotinsäureamid; arithmetische Mittel ± SEM (n = 9) der (A) Nahrungs- (g/24 h) und (B) Flüssigkeitsaufnahmen (ml/24 h) sowie (C) das Körpergewicht (g) vor (unausgefüllte Balken) und nach (ausgefüllte Balken) der Verabreichung von Nicotinsäureamid (0,5 mg/g), das dem Trinkwasser hinzugegeben wurde. * zeigt einen statistisch signifikanten (p < 0,05) Unterschied im gepaarten Vergleich mit dem jeweiligen Wert vor der Verabreichung von Nicotinsäureamid an;

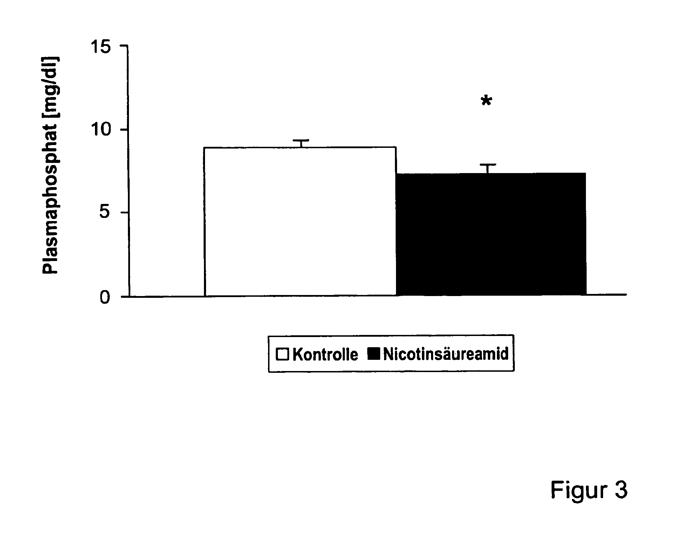

- Fig. 3: zeigt die Plasmaphosphatkonzentration vor und nach der Verabreichung von Nicotinsäureamid; arithmetische Mittel arithmetische Mittel ± SEM (n = 9) der Phosphatkonzentration im Plasma (mg/dl) vor (unausgefüllte Balken) und nach (ausgefüllte Balken) der Verabreichung von Nicotinsäureamid (0,5 mg/g), das dem Trinkwasser hinzugegeben wurde. * zeigt einen statistisch signifikanten (p < 0,05) Unterschied im gepaarten Vergleich mit dem jeweiligen Wert vor der Verabreichung von Nicotinsäureamid an;

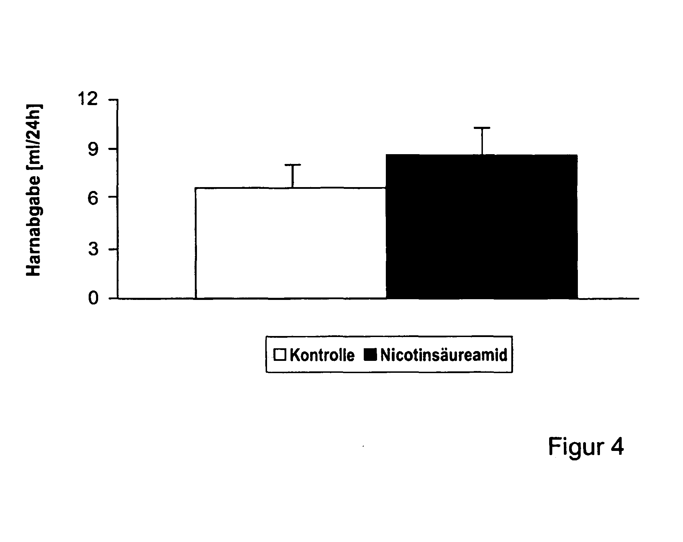

- Fig. 4: zeigt die Harnflussrate vor und nach der Verabreichung von Nicotinsäureamid; arithmetische Mittel arithmetische Mittel ± SEM (n = 9) der Harnflussrate (µl/24 h) vor (unausgefüllte Balken) und nach (ausgefüllte Balken) der Verabreichung von Nicotinsäureamid (0,5 mg/g), das dem Trinkwasser hinzugegeben wurde;

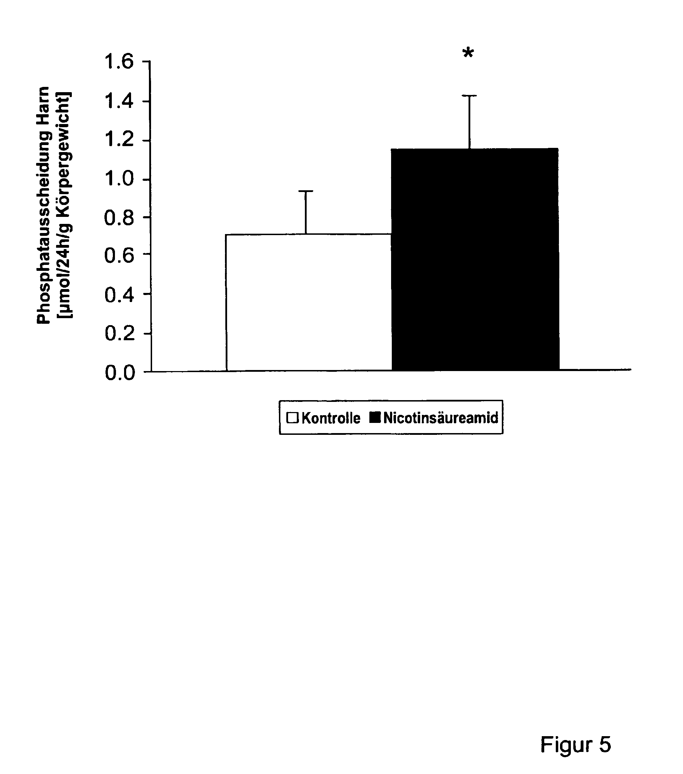

- Fig. 5: zeigt die Phosphatausscheidung über den Harn vor und nach der Verabreichung von Nicotinsäureamid; arithmetische Mittel plus minus SEM (n = 9) der Exkretion über den Harn von (A) Phosphat (µg pro 24 h/g Körpergewicht) vor (unausgefüllte Balken) und nach (ausgefüllte Balken) der Verabreichung von Nicotinsäureamid (0,5 mg/g), das dem Trinkwasser zugesetzt wurde. * zeigt einen statistisch signifikanten (p < 0,05) Unterschied im gepaarten Vergleich zu dem jeweiligen Wert vor der Verabreichung von Nicotinsäureamid an;

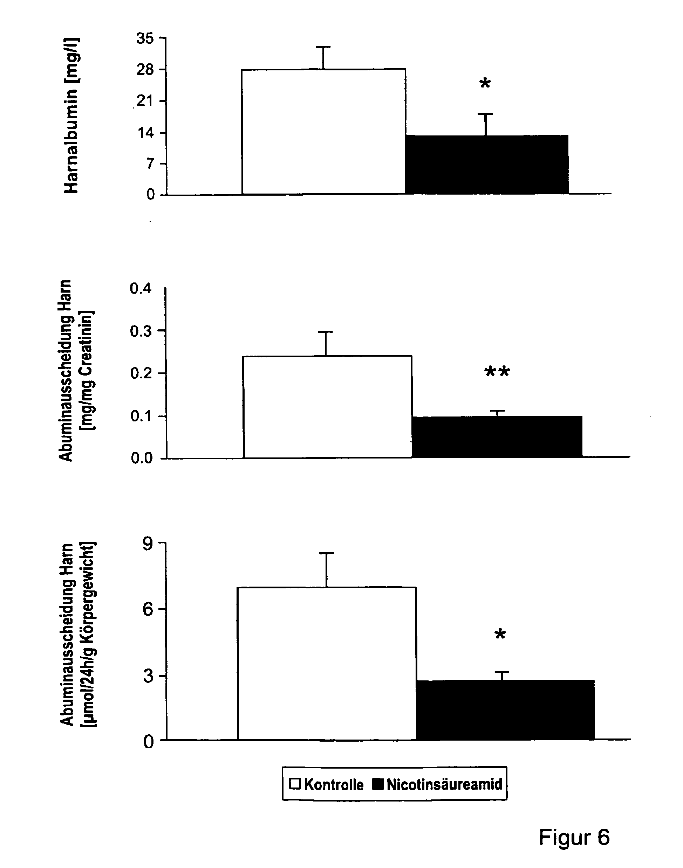

- Fig. 6: zeigt die Ausscheidung von Albumin über den Harn vor und nach der Verabreichung von Nicotinsäureamid; arithmetische Mittel plus minus SEM (n = 9) der Ausscheidung von Albumin über den Harn (A. µg pro 24 h oder B. mg/mg Creatinin oder C. Mikromol/24 h/g Körpergewicht) vor (unausgefüllte Balken) und nach (ausgefüllte Balken) der Verabreichung von Nicotinsäureamid (0,5 mg/g), das dem Trinkwasser zugegeben wurde. * zeigt einen statistisch signifikanten (p < 0,05) Unterschied im gepaarten Vergleich zu dem jeweiligen Wert vor der Verabreichung von Nicotinsäureamid an.

### Ausführungsbeispiele

### 1. Material und Methoden

### 1.1 Tierversuche

Sämtliche Tierversuche wurden gemäß der Richtlinien der Amerikanischen Physiologischen Gesellschaft und in Übereinstimmung mit dem deutschen Tierschutzgesetz durchgeführt und von den lokalen Behörden genehmigt.

Die Experimente wurden an 6 bis 10 Monate alten Akita-Mäusen durchgeführt, die spontan Diabetes vom Typ I entwickeln; Dreyer et al.: Diabetic nephropathy: leveraging mouse genetics. Curr. Opin. Nephrol. Hypertens. 2006; 15: 227-232. Die Tiere wurden bei einer Kontrolldiät gehalten (C1314 - 0,2 % Na⁺, 1 % K⁺, 0,9 % Ca⁺⁺, Altromin, Heidenau, Deutschland) und hatten während der Experimente freien Zugang zu Trinkwasser. Sofern angegeben, wurde dem Trinkwasser Nicotinsäureamid (0,5 mg/g) zugegeben. Zur Bewertung der renalen Ausscheidung und Sammlung von Harn über 24 Stunden wurden die Mäuse individuell in Stoffwechselkäfigen (Tecniplast, Hohenpeißenberg, Deutschland) platziert, wobei der freie Zugang zu Trinkwasser gegeben war. Die innere Wand der Stoffwechselkäfige war silikonisiert und der Harn wurde unter wassergesättigtem Öl gesammelt.

Um Blutproben zu erhalten, wurden die Tiere mit Isofluran (Abbott, Wiesbaden, Deutschland) leicht anästhetisiert und durch Punktieren des retroorbitalen Plexus wurden etwa 200 µl Blut in heparinisierte Kapillaren entnommen.

Gesammelter Kot wurde bei etwa 80°C für etwa 3 Stunden getrocknet und anschließend wurde das Kot-Trockengewicht bestimmt. Zu dem Kot wurden 5 ml von 0,75 M HNO₃ hinzugegeben und die Probe wurde für 48 Stunden auf einem elektrischen Schüttler geschüttelt. Die Proben wurden dann bei 3.500 rpm für 10 Minuten zentrifugiert und 1 ml des Überstandes wurde gesammelt. Der Überstand wurde erneut bei 14.000 rpm für 5 Minuten zentrifugiert. Der Überstand wurde bei -20°C bis zur Analyse gelagert. Der Überstand wurde analysiert, wie unten beschrieben.

### 1.2 Konzentrationsmessungen im Plasma und im Harn

Die Konzentrationen von Na⁺ und K⁺ im Plasma und im Harn wurden mittels Flammenfotometrie (ELEX 6361, Eppendorf, Deutschland) gemessen. Die Konzentrationen von Phosphat und Ca⁺⁺ wurden kolorimetrisch unter Verwendung eines kommerziellen Diagnostikkits (Rohrsteig Nostiscore Mannheim, Deutschland) bestimmt. Die Konzentrationen von Cl⁻ wurden mittels elektrometrischer Titration (Chloridometer 6610, Eppendorf, Deutschland) analysiert.

Die Konzentrationen von Glucose im Plasma und im Harn wurden unter Verwendung eines Glucometers (Accutrend, Bosch, Mannheim, Deutschland) bestimmt. Die Konzentrationen von Creatinin im Serum wurden unter Verwendung eines enzymatischen Kits (Demeditec, Kiel, Deutschland), und im Harn unter Verwendung der Jaffe-Reaktion (Sigma, St. Louis, USA) gemäß den Angaben der Hersteller gemessen.

### 1.3 Bestimmung der Albuminkonzentrationen im Harn

Die Albuminkonzentrationen im Harn wurden fluorometrisch unter Verwendung des albuminsensitiven Farbstoffs Albumin Blau 580 nach einer Anregung bei 600 nm und bei einer Emission bei 645 nm gemäß den Angaben des Herstellers (microfluoral, Progen, Heidelberg, Deutschland) gemessen. Es wurden Standardkurven mit Maus-Albumin (Sigma, Taufkirchen, Deutschland) generiert, und die Messungen wurden innerhalb des linearen Bereichs von 0 bis 156 mg/l durchgeführt.

### 1.4 Herkunft der Substanzen

Sämtliche Substanzen stammten von Sigma (Taufkirchen, Deutschland) oder Roth (Karlsruhe, Deutschland)

### 1.5 Statistik

Die Daten werden als Mittelwerte ± SEM dargestellt, n stellt die Anzahl von unabhängigen Experimenten dar. Sämtliche Daten wurden unter Verwendung des ungepaarten Student's t-Test mit Welch-Korrektur auf Signifikanz getestet, sofern erforderlich.

### 2. Ergebnisse

### 2.1 Glucosekonzentration im Plasma und Ausscheidung von Glucose über den Harn

Wie erwartet, entwickelten die Akita-Mäuse innerhalb weniger Wochen Diabetes mellitus. Die Glucosekonzentrationen im Blut lagen nahe bei 20 mmol pro Liter; vgl. Fig. 1. Die Hyperglycämie erhöhte die filtrierte Glucoseladung über die maximale Transportrate, was zu einer Glucosurie führte.

Die Verabreichung von Nicotinsäureamid (0,5 mg Körpergewicht) zeigte auf die Konzentration von Glucose im Plasma nur eine geringe Wirkung (Fig. 1a), wohingegen die Ausscheidung von Glucose über den Harn signifikant zunahm; vgl. Fig. 1b.

### 2.2 Flüssigkeits- und Nahrungsaufnahme, Körpergewicht

Die Verabreichung von Nicotinsäureamid führte zu einer geringen, jedoch signifikanten Zunahme der Nahrungs- (Fig. 2a) und Flüssigkeitsaufnahme (Fig. 2b), zeigte jedoch keine Auswirkung auf das Körpergewicht (Fig. 2c).

### 2.3 Konzentration von Phosphat im Plasma

Die Verabreichung von Nicotinsäureamid führte zu der erwarteten signifikanten Abnahme der Konzentration von Phosphat im Plasma; vgl. Fig. 3.

### 2.4 Harnausscheidung

Die Harnausscheidung tendierte nach der Behandlung mit Nicotinsäureamid zu einer leichten Zunahme (Fig. 4a), wobei diese Wirkung keine statistische Signifikanz erreichte.

### 2.5 Ausscheidung von Phosphat über den Harn

Die Behandlung mit Nicotinsäureamid führte trotz der Abnahme der Phosphatkonzentration im Plasma zu einer signifikanten Zunahme der renalen Phosphatausscheidung (Fig. 5).

### 2.6 Hemmung der Proteinurie

Die Proteinurie wurde durch die Behandlung mit Nicotinsäureamid signifikant abgeschwächt. Die Konzentration von Album im Harn wurde durch die Verabreichung von Nicotinsäureamid um über 50 % reduziert; vgl. Fig. 6A. Die Ausscheidung von Albumin über der Harn wurde um über 60 % reduziert; vgl. Fig. 6B (in Bezug auf die Creatininkonzentration, um die unterschiedliche Harnkonzentrierung zu normieren) und 6C (pro 24 Stunden und pro Körpergewicht).

### 3. Fazit

Die Erfinder konnten anhand von Tierversuchen nachweisen, dass die bei einer Proteinurie beobachtete verstärkte Ausscheidung von Proteinen über den Harn durch die Verabreichung von Nicotinsäureamid reduziert wird. Bei Nicotinsäureamid handelt es sich deshalb um eine neue Substanz zur Behandlung und/oder Prävention von Proteinurie.

## Patentansprüche

1. Verwendung von Nicotinsäureamid zur Herstellung eines Arzneimittels zur Behandlung und/oder Prävention von Proteinurie, wobei das Nicotinsäureamid die einzige gegen Proteinurie wirksame Substanz ist.

2. Verwendung nach Anspruch 1, **dadurch gekennzeichnet, dass** die Behandlung und/oder Prävention von Proteinurie bei einem Patienten erfolgt, der unter einer Krankheit/Störung leidet oder eine Veranlagung für eine Krankheit/Störung aufweist, die ausgewählt ist aus der Gruppe bestehend aus: chronisches Nierenversagen, Nierenentzündung, Harnleiterentzündung, Blasenentzündungen, Diabetes mellitus, arterielle Hypertonie, Medikamentenabhängigkeit, EPH-Gestose, erhöhte/erniedrigte Körpertemperatur, erhöhte Harnsäurewerte (Gicht), multiples Myelom, Störungen der Harnblasenentleerung mit Rückstau in der Niere, Hypokaliämie, Hypercalcämie, Hämolyse, Myoglobinämie, Tumoren, Plasmocytom mit der Entstehung von Bence-Jones-Proteinen, Harnleitersteine, Blasensteine.

3. Verwendung nach Anspruch 1 oder 2, **dadurch gekennzeichnet, dass** das Arzneimittel für eine Applikation ausgebildet ist, die ausgewählt ist aus der Gruppe bestehend aus: oral, rektal, parenteral, lokal, transdermal.

4. Verwendung nach einem der Ansprüche 1 bis 3, **dadurch gekennzeichnet, dass** das Arzneimittel in einer Form ausgebildet ist, die ausgewählt ist aus der Gruppe bestehend aus: Pulver, Tablette, Saft, Tropfen, Kapsel, Zäpfchen, Lösung, Injektionslösung, Aerosol, Salbe, Spülung, Pflaster, Pellet, Dragee, modifiziert freisetzende Darreichungsform.

5. Verwendung nach einem der Ansprüche 1 bis 4, **dadurch gekennzeichnet, dass** das Arzneimittel Nicotinsäureamid in einer Konzentration aufweist, die ausgewählt ist aus der Gruppe bestehend aus (jeweils angegeben in Gewicht des Wirkstoffs Nicotinsäureamid bezogen auf das Gesamtgewicht des Arzneimittels): 1 µg/g, 10 µg/g, 100 µg/g, 1 mg/g, 10 mg/g, 100 mg/g, 1 g/g.

6. Verwendung nach einem der Ansprüche 1 bis 5, **dadurch gekennzeichnet, dass** die Absolutmenge des Wirkstoffs Nicotinsäureamid in einer Dosierungseinheit des Arzneimittels zwischen ca. 1 und ca. 3000 mg, vorzugsweise zwischen ca. 10 und ca. 1500 mg, weiter bevorzugt zwischen ca. 100 und ca. 750 mg, und höchst bevorzugt bei ca. 500 mg liegt.

## Claims

1. Use of nicotinic acid amide for the production of a medicament for the treatment and/or prevention of proteinuria, wherein the nicotinic acid amide is the only substance being effective against proteinuria.

2. Use of claim1, **characterized in that** the treatment and/or prevention of proteinuria is effected on a patient suffering from a disease/disorder or having a disposition for a disease/disorder which is selected from the group consisting of: chronic renal failure, renal inflammation, ureter inflammation, inflammation of the bladder, diabetes mellitus, arterial hypertension, medicine dependency, EPH gestosis, increased/decreased body temperature, increased urine acid values (gout), multiple myeloma, dysfunction of the bladder emptying with renal reflux, hypokalaemia, hypocalcaemia, hemolysis, myoglobinaemia, tumors, plasmocytome with the development of Bence-Jones-proteins, ureter stones, bladder stones.

3. Use of claim 1 or 2 **characterized in that** the medicament is configured for an application which is selected from the group consisting of: oral, rectal, parenteral, local, transdermal.

4. Use of any of the claims 1 to 3 **characterized in that** the medicament is configured in a form which is selected from the group consisting of: powder, tablet, syrup, drops, capsule, suppository, solution, injection solution, aerosol, ointment, rinse, plaster, pellet, dragée, modified releasing dosage form.

5. Use of any of the claims 1 to 4 **characterized in that** the medicament comprises nicotinic acid amide in a concentration which is selected from the group consisting of (in each case indicated as weight of the active agent nicotinic amide relating to the total weight of the medicament): 1µg/g, 10 µg/g, 100 µg/g, 1 mg/g, 10 mg/g, 100mg/g, 1g/g.

6. Use of any of the claims 1 to 5, **characterized in that** the absolute quantity of the active agent nicotinic acid amide in a dosage unit of the medicament is between approx. 1 and approx. 3000 mg, preferably between approx. 10 and approx. 1500 mg, further preferred between approx. 100 and approx. 750 mg and highly preferred approx. 500 mg.

## Revendications

1. Utilisation d'amide d'acide nicotinique pour la production d'un médicament pour le traitement et/ou la prévention de la protéinurie, l'amide d'acide nicotinique étant la seule substance efficace contre la protéinurie.

2. Utilisation selon la revendication 1, **caractérisée en ce que** le traitement et/ou la prévention de la protéinurie s'effectue chez un patient qui souffre d'une maladie/ d'un trouble ou d'une prédisposition pour une maladie/un trouble, qui est choisi(e) dans le groupe comprenant une insuffisance rénale chronique, une inflammation rénale, une inflammation des voies urinaires, des inflammations de la vessie, le diabète sucré, l'hypertension artérielle, la dépendance médicamenteuse, la gestose EPH, une augmentation/ diminution de la température corporelle, une augmentation de l'acide urique (goutte), le myélone multiple, les troubles de la miction avec rétention rénale, une hypokaliémie, une hypercalcémie, une hémolyse, une myoglobinélie, des tumeurs, un plasmocytome avec développement de protéines de Bence-Jones, des calculs des voies urinaires, les calculs vésicaux.

3. Utilisation selon la revendication 1 ou 2, **caractérisée en ce que** le médicament est conçu pour une application qui est choisie dans le groupe comprenant les voies orale, rectale, parentérale, locale, transdermique.

4. Utilisation selon l'une des revendications 1 à 3, **caractérisée en ce que** le médicament est conçu sous une forme qui est choisie dans le groupe comprenant une poudre, un comprimé, un jus, des gouttes, des capsules, des suppositoires, une solution, une solution pour injection, un aérosol, une pommade, un rinçage, un pansement, une pastille, une dragée, une forme galénique à libération modifiée.

5. Utilisation selon l'une des revendications 1 à 4, **caractérisée en ce que** le médicament présente un amide d'acide nicotinique en une concentration qui est choisie dans le groupe comprenant (respectivement en poids de substance active amide d'acide nicotinique par rapport au poids total de médicament) : 1 µg/g, 10 µg/g, 100 µg/g, 1 mg/g, 10 mg/g, 100 mg/g, 1 g/g.

6. Utilisation selon l'une des revendications 1 à 5, **caractérisée en ce que** la quantité absolue de substance active amide d'acide nicotinique dans une unité galénique du médicament se situe entre environ 1 et environ 3000 mg, de préférence entre environ 10 et environ 1500 mg, de manière davantage préférée, entre environ 100 et environ 750 mg et de manière préférée entre toutes, est d'environ 500 mg.
